# EUROPEAN PATENT APPLICATION

(11) **EP 1 923 416 A1**
(43) Date of publication of application: **21.05.2008**
(21) Application number: 06797202.6
(22) Date of filing: 31.08.2006
(51) Int. Cl.: C08G 59/24, C07D 301/00, C07D 303/27, C08G 65/18, H01L 23/29, H01L 23/31

(54) **ADAMANTANE DERIVATIVE, EPOXY RESIN, AND OPTICAL ELECTRONIC MEMBER USING RESIN COMPOSITION COMPRISING THEM**

(30) Priority: 05.09.2005 JP 2005256795
(71) Applicant: Idemitsu Kosan Co., Ltd., Chiyoda-ku Tokyo 100-8321 (JP)
(72) Inventor: ITO, Hajime, Ichihara-shi, Chiba 299-0193 (JP)
(74) Representative: Vossius & Partner
(86) International application number: PCT/JP2006/317242
(87) International publication number: WO 2007/029598

(57) **Abstract**

This invention provides an adamantane derivative that can provide a cured product having optical properties such as transparency and lightfastness, long-term resistance, electric characteristics such as permittivity, and an epoxy resin having an adamantane skeleton, a composition comprising them, and an optical electronic member using the resin composition. The adamantane derivative is represented by general formula (I). The epoxy resin having an adamantane skeleton is represented by general formula (V) or (VI). The composition comprises them. The optical electronic uses the resin composition. In the formula, W represents, for example, a hydrogen atom, X represents a group represented by general formula (II), Y represents, for example, -O-, Z represents a group represented by formula (III) or (IV), R¹ represents a methyl or ethyl group, R² represents an alkyl group having 1 to 4 carbon atoms, k is an integer of 0 to 10, m is an integer of 0 to 14, n is an integer of 2 to 16, m + n = 16, and p and q are an integer of 0 to 5.

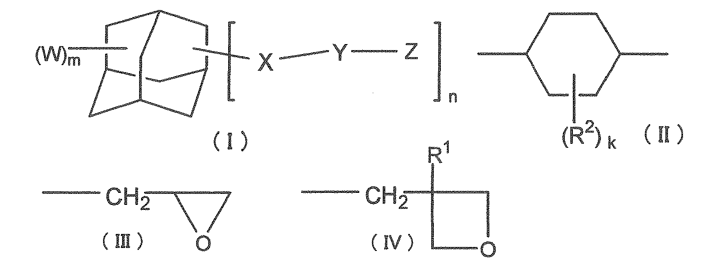

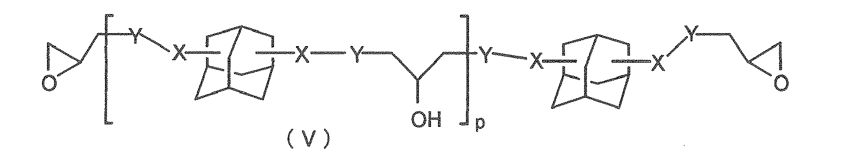

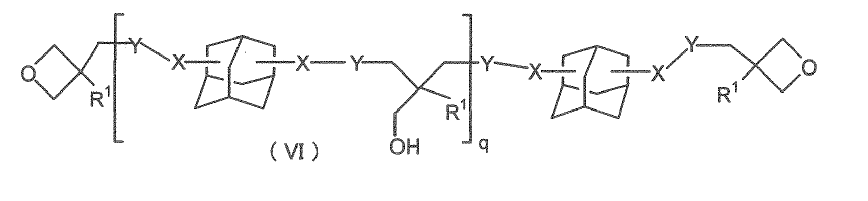

## Description

### Technical Field

The present invention relates to a novel adamantane derivative, a novel epoxy containing an adamantane skeleton, and an optical electronic member using a composition comprising them, and specifically to a resin composition as a sealant for an electronic circuit (an optical semiconductor, an organic electroluminescence (EL) device the like), as an optical electronic member (an optical waveguide, an optical communication lens, an optical film and the like) and others, and an optical electronic member the resin composition thereof

### Background Art

An adamantane contains 4 cyclohexane rings to form a skeleton, and is highly symmetric and stable. Its derivatives show specific performances, thus are known to be as raw materials for a pharmaceutical raw material, a high performance industrial material and the like. An adamantane has, for example, optical characteristics, heat resistance the like, and therefore attempts have been made to use it for an optical disk substrate, an optical fiber, a lens and the like (for example, refer to Patent Documents 1 and 2). Furthermore, there have been attempts to use an adamantane as a raw resin material for a photoresist by utilizing its acid-sensitive property, dry etching resistance, UV light transparency and the like (for example, refer to Patent Document 3).
In recent years, in order to achieve higher performance or improvement of an optical/electronic component, are progressing for higher precision, wider viewing angle, and enhanced image quality of a flat display using a liquid crystal, an organic EL device and the like, for higher intensity/shorter wavelength and whitening of a light source using such optical semiconductors as a light emitting diode (LED) and the like, further for higher frequency of an electronic circuit and for an optical circuit/communication, and others.
As a method for the improvement, a basic material of a light emitting material and the like used for a liquid crystal material and an organic EL device has been investigated and developed. The investigation has also been done to higher performance of a resin that is used along with those materials as a coating material, a and the like. As a resin for a coating material of an optical/electronic component and for a sealant, many kinds of thermosetting resin, light-curable resin, or thermoplastic resin have been applied. They have been applied in accordance with their respective characteristics in heat transparency, solubility, adhesiveness, and others of each resin.
In the field of LED, which is advanced in terms of high performance, an illumination, a light and the like using a white LED comprising emitting devices of ultra violet and blue have proposed and developed for practical use. In addition, it is expected they will be developed to be for a home lighting and an automobile in the future. An LED device is by a resin containing a fluorescent material in an inorganic semiconductor. For such applications, thermosetting resins such as conventional bisphenol A epoxy and the like have limitation in resistance and light resistance, thus a fulfilling those required characteristics is desired (for instance, refer to Non-Patent Document 1). As its improved product, a hydrogenated alicyclic epoxy compound and the like having low absorption in a shorter wavelength have proposed, but their heat resistance is lowered on the contrary.

Furthermore, in the display field, an organic EL device of small size, high precision, and energy saving is used, and such type as top-emission is employed. Accordingly, a sealing resin itself is required, for the use in an organic EL device, to have further improved transparency, light resistance, heat-resistance, mechanical strength and others in addition to functions such as gas barrier, adhesion of conventional sealing boards such as stainless steel and the like to a glass substrate, and others (for instance, refer to Non-Patent Document 2).
Furthermore, in an electronic circuit with a semiconductor and the like, as a computerized society progresses, the increase in volume of information and communication speed and the miniaturization of a device have been achieved, thus further miniaturization, integration, and an increase in frequency are necessary. Furthermore, an optical circuit using an optical waveguide and the like that enable further high speed processing has also investigated. Such conventional resins as a bisphenol A epoxy resin the like, which have been used as a resin, a film for these uses, or a for a lens, have dielectric constant in an electronic circuit, and a decrease in and turn to yellow due to decaying by light absorption due to an aromatic ring in an waveguide and an LED and so on.
For these reasons, has been used an alicyclic epoxy resin having no aromatic ring which is prepared by such methods as epoxidizing a carbon-carbon double bond, reacting a polyvalent alicyclic alcohol with epichlorohydrin, hydrogenating an aromatic ring of an aromatic epoxy resin and the like. However, a cured cyclic aliphatic epoxy resin (such as 3,4-epoxycyclohexylmethyl-3',4'-epoxycyclohexanecarboxylate and the like) that is prepared by epoxidizing a carbon-carbon double bond is brittle and insufficient in impact strength and in adhesion strength to a metal and the like. Also, as an alicyclic epoxy resin that is prepared by reacting a polyvalent alicyclic alcohol with epichlorohydrin or by hydrogenating an aromatic ring of an aromatic epoxy resin, epoxy resins containing hydrogenated bisphenol A or bisphenol F are known (for instance, refer to Patent Document 4), but they are inferior to a bisphenol epoxy resin in resistance. Also, in an epoxy resin that is prepared by reacting a hydrogenated bisphenol A with epichlorohydrin there have been problems of deterioration of electric characteristics such as an increase in dielectric constant and the like since a chlorine component tended to remain in the product during its production.
On the other hand, a polymer compound having an adamantane skeleton is excellent in heat and, for instance, a polyester, a polycarbonate and the like, all using an adamantane diet, are known as such polymers. Also, as a resin composition using an adamantane, a resin composition containing 1,3-bis(glycidyloxyphenyl)adamantane (for instance, refer to Patent Document 5) and a composition containing 2,2-bis(glycidyloxyphenyl)adamantane (for instance, refer to Document 6) are disclosed. Although lowering of dielectric and improvement of transparency have observed in these compositions as compared with a bisphenol A epoxy resin, it may not be said that these are sufficient since these adamantanes an aromatic ring. In addition, since these adamantanes are crystalline compounds with high melting points, heating was necessary when mixing them with a curing agent, resulting in a problem of short workable time.

Patent Document 1: Japanese Patent Laid-Open Publication No. H6-305044
Patent Document 2: Japanese Patent Laid-Open Publication No. H9-302077
Patent Document 3: Japanese Patent Laid-Open Publication No. H4-39665
Non-Patent Document 1: Monthly "Material Stage" June 2003, pp.20-24, published by Technical Information Institute Co., Ltd.
Non-Patent Document 2: Monthly "Material Stage" March 2003, pp. 52-64, published by Technical Information Institute Co., Ltd.
Patent Document 4: Japanese Patent Laid-Open Publication No. 2000-143939
Patent Document 5: Japanese Patent Laid-Open Publication No. 2003-321530
Document 6: Japanese Laid-Open Publication No. H10-130371

### Disclosure of the Invention

### Problems to be Solved by the Invention

In view of the above-mentioned circumstances, an object of the present invention is to provide an adamantane derivative giving a cured product that is excellent in optical characteristics such as transparency and light resistance, long-term resistance, and electric characteristics such as dielectric constant and the like, useful as a for an electronic circuit (sealants for an optical semiconductor, an organic EL device and the like), as an optical electronic member (an optical waveguide, a lens for optical communication, an optical film and the like), and as an adhesive for these. And also, an object of the present invention is to provide an epoxy resin having an adamantane skeleton, a resin composition containing them, and an optical electronic member using the resin composition.

### Means for Solving the Problems

The present inventors investigated extensively to achieve the above objectives, and as a result, have found that a resin composition giving a cured product suitable as an optical electronic member could be obtained by using a specific adamantane derivative an epoxy resin having a specific adamantane skeleton. The present invention was accomplished based on these findings.
Namely, the present invention provides a following adamantane derivative, an epoxy resin containing an adamantane skeleton, a resin composition containing them, and an optical electronic member using the resin composition.
1. An adamantane derivative represented by the following formula (I).

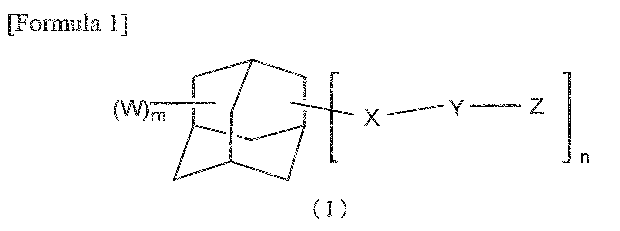

[In the formula, W represents a group selected from a hydrogen atom, a halogen atom, a hydrocarbon group, a hydrocarbon group, a cyclic hydrocarbon group, a halogen-substituted cyclic hydrocarbon group, a hydroxyl group, a carboxyl group, and =O formed from two Ws combined together. X represents a group represented by the following formula (II).

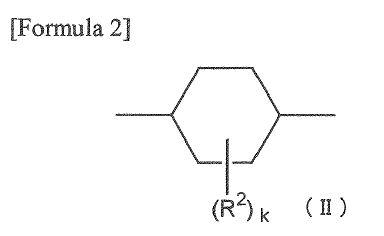

(In the formula, R² represents an alkyl group having 1 to 4 carbon atoms.) Y represents a group selected from -CO₂-, -O-, -N(R³)- (R³ represents a hydrogen atom or an alkyl group having 1 to 4 carbon atoms), and -N(Z)-. Z represents a group represented by the following formula (III) or (IV).

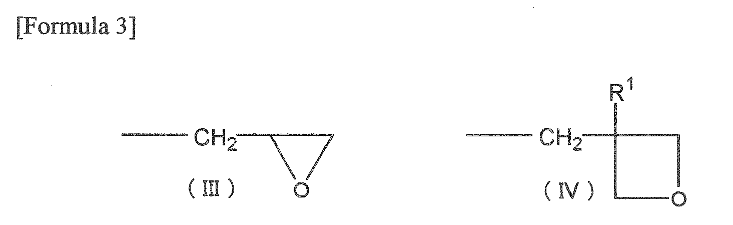

(In the formula, R¹ represents a methyl group or an ethyl group), k represents an integer of 0 to 10, m represents an integer of 0 to 14, n represents an integer of 2 to 16, and m + n= 16]
2. The adamantane derivative according to the above 1, wherein X is bonded to the bridgehead position of the adamantane skeleton and n is 2 to 4 in the formula (I).
3. The adamantane derivative according to the above 1, wherein n is 2 and X is bonded to the same methylene position of the skeleton in the formula (I).
4. The adamantane derivative according to the above 2 or 3, wherein Y is -O- in the formula (I).
5. The adamantane derivative according to any of the above 1 to 4, wherein the content of hydrolyzable chlorine is 500 pp by mass or less.
6. An epoxy resin having an adamantane skeleton represented by the following general formula (V) or (VI).

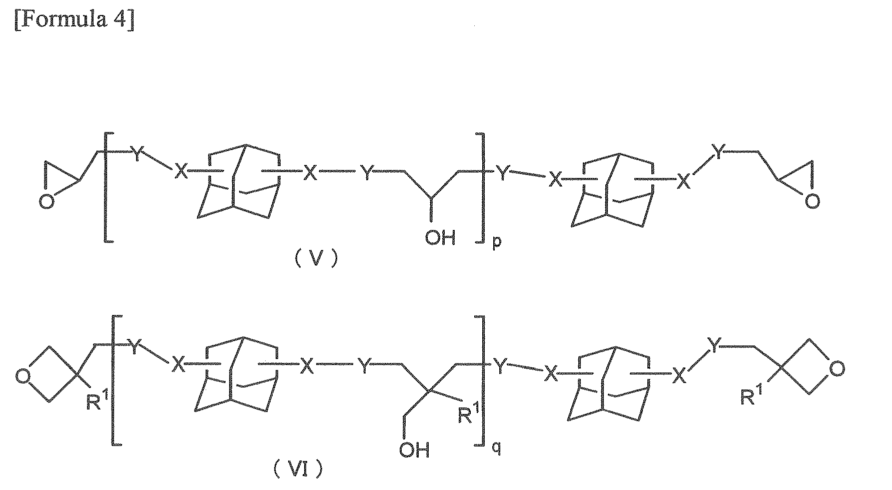

### [In the formula, X, Y, and 1 are the same as before, and p and q are an integer of 0 to 5]

7. The epoxy resin according to the above 6, wherein the content of hydrolyzable chlorine is 500 pp by or less.
8. A method for producing the adamantane derivative according to any of the above 1 to 5, wherein a corresponding aromatic adamantane derivative is ring-hydrogenated in the presence of a rhodium catalyst or a ruthenium catalyst.
9. A resin composition containing the adamantane derivative according to any of the above I to 5 and/or the epoxy resin according to the above 6 or 7, and a curing for an epoxy resin.
10. The resin composition according to the above 9, wherein the curing for an epoxy resin is a cationic polymerization initiator and/or an acid anhydride type curing agent.
11. An optical electronic member using the resin composition according to the above 9 or 10.
12. A sealant for an electronic circuit using the resin composition of the above 9 or 10.

### Effects of the Invention

A resin composition containing an adamantane derivative and/or an epoxy resin having an adamantane skeleton of the invention gives a cured product which is excellent in optical characteristics such as transparency light resistance, long-term resistance, electric characteristics such as dielectric constant, and is suitable as a sealant for an electronic circuit (a for an optical semiconductor, a sealant for an organic EL device and the like), as an optical electronic member (an optical waveguide, a lens for optical communication, an optical film and the like), and as an adhesive for them.

### Mode for Carrying Out the Invention

An adamantane derivative of the present invention is represented by the following general formula (I).

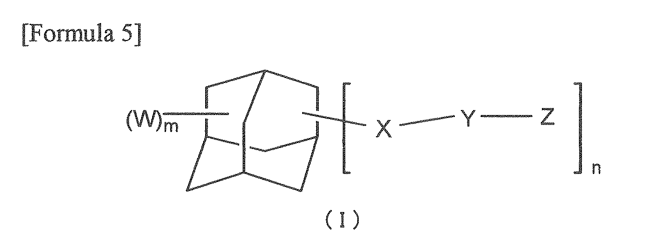

In the formula, W represents a group selected from a hydrogen atom, a halogen atom, a hydrocarbon group, a halogen-substituted hydrocarbon group, a cyclic hydrocarbon group, a halogen-substituted cyclic hydrocarbon group, a hydroxyl group, a carboxyl group, and =O formed from two Ws combined together. Preferable examples of the hydrocarbon groups represented by W include, for instance, an alkyl group having 1 to 10 carbon atoms, an alkoxy group and the like. Any of linear, branched, or cyclic alkyl group may be used. Specific examples include such groups as a methyl group, an ethyl group, a propyl group, a butyl group, a cyclohexyl group and the like. Examples of the alkoxy include a methoxy group, an ethoxy group and the like. Examples of the halogen-substituted hydrocarbons include the above-mentioned hydrocarbon groups in which one or more of hydrogen atom(s) is (are) replaced by (a) halogen atom(s), such groups as a trifluoromethyl group and the like. Examples of the halogen atoms are fluorine, chlorine, bromine, and iodine.
Examples of the cyclic hydrocarbon groups represented by W include a cycloalkyl group having 5 to 10 carbon atoms, specifically such groups as a cyclopentyl group, a methylcyclopentyl group, a cyclohexyl group, a methylcyclohexyl group, an ethylcyclohexyl group and the like. Further, examples of the halogen-substituted cyclic hydrocarbon groups are the above-mentioned cyclic hydrocarbon group in which one or more of hydrogen atom(s) is (are) replaced by (a) halogen atom(s), for instance, they include such groups as a flurorocyclopentyl group, a fluorocyclohexyl group, a trifluoromethylcyclopentyl group, a trifluoromethylcyclohexyl group and the like.

In the above general formula (1), X represents a group represented by the following formula (II).

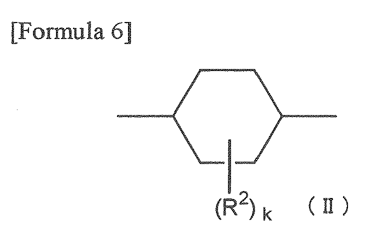

In the formula, R² represents an alkyl group having 1 to 4 carbon atoms. Examples of the alkyl group having 1 to 4 carbon atoms include a ethyl group, an ethyl group, any propyl groups, and any butyl groups. Plural R² may be the same or different.
In the above formula (I), Y represents a group selected from -CO₂-, -O-, -N(R³)- (R³ represents a hydrogen atom or an alkyl group having 1 to 4 carbon atoms), and -N(Z)-. Z represents a group represented by the following formula (III) or (IV). An alkyl group having 1 to 4 atoms in R³ is the same as those for R₂.

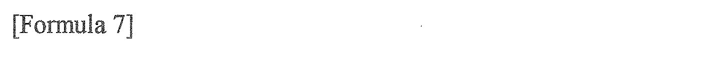

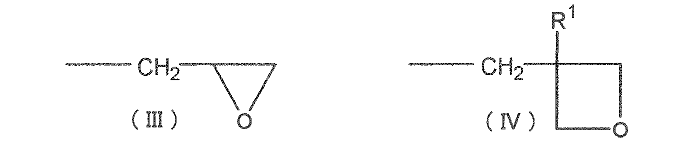

In the formula, R¹ represents a methyl group or an ethyl group, and k represents an integer of 0 to 10. In the above general formula (1), m represents an integer of 0 to 14, n represents an integer of 2 to 16, and + n = 16.
Examples of the adamantane derivatives of the present invention include, for example, in the general formula (I), an adamantane derivative in which X is bonded to the bridgehead of the adamantane skeleton and n is 2 to 4, an adamantane derivative in which n is 2 and X is bonded to the methylene position of the adamantane skeleton, an adamantane derivative in which Y represents -O- in these derivatives and the like. In an adamantane derivative represented by the above general formula (I), it is preferable that the content of hydrolyzable chlorine should be 500 ppm by mass or less.
An adamantane derivative represented by the above formula (I) is a non-aromatic derivative, wherein an adamantane having excellent heat resistance and transparency is with a cyclic group via a linking group containing a cyclohexyl structure. By such a structure, light resistance, dielectric constant and the like as well as heat resistance transparency are improved solubility for practical use is also imparted.
As for synthesizing an derivative represented by the above formula (I), a method of reacting a corresponding adamantane-containing carboxylic derivative or an adamantane-containing alcohol derivative with epichlorohydrin the like, and a method of ring-hydrogenating a corresponding aromatic adamantane derivative are known, but in order to reduce the content of hydrolyzable chlorine in the non-aromatic adamantane derivative obtained, the latter synthesis method is preferable. Examples of the synthesis methods of the adamantane derivatives represented by the above general formula (I) include the following synthesis methods 1 to 3.
Synthesis method 1 is, for instance, to hydrogenate adamantanebisphenol glycidyl ethers such as 1,3-bis(4-glycidyloxyphenyl)adamantane, 2,2-bis(4-glycidyloxypheyl)adamantane and the like, as shown in the following general formulas (i-1) and (i-2) to obtain a non-aromatic adamantane derivative.

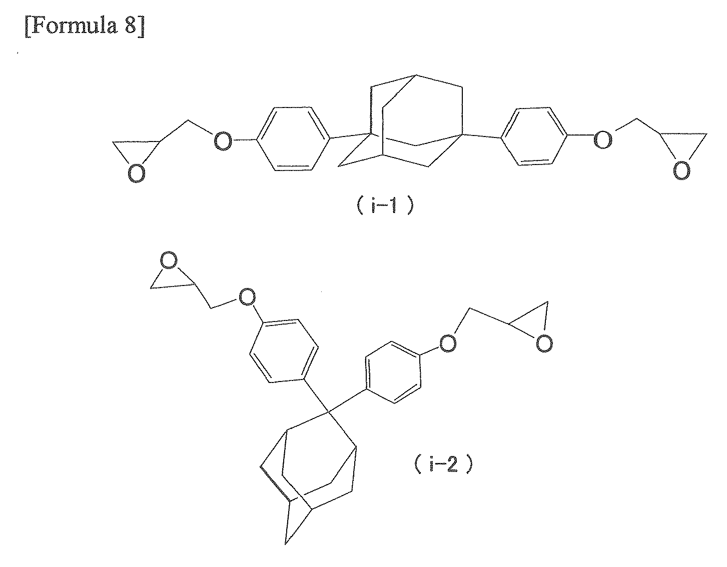

The reaction temperature of the above hydrogenation is usually about 20 to about 150°C, and preferably 40 to 100°C. When the reaction temperature is 20°C or above, the reaction rate does not decrease and remains moderate, thus the reaction time is shortened. And, when the reaction temperature is 150°C or below, hydrogenation of the intended derivative is suppressed. Applied hydrogen pressure at the reaction is about 1 to about 30 MPa, and preferably 3 to 10 MPa. When the reaction pressure is 30 MPa or less, special equipment is not necessary as the safety is secured, thus it is useful from an industrial viewpoint. The reaction time is usually about 1 minute to about 24 hours, and preferably 1 to 10 hours.

The above reaction is carried out usually in the presence of a rhodium catalyst or a ruthenium catalyst. As the rhodium catalyst, examples include rhodium supported on activated carbon or alumina, a rhodium oxide and the like. As the ruthenium catalyst, examples include ruthenium supported on activated carbon or alumina, a ruthenium oxide, ruthenium black and the like.
The ratio of a rhodium catalyst or a ruthenium catalyst used, in terms of the amount of rhodium or ruthenium, is about 0.01 to about 10% by mass, preferably 0.05 to 5% by mass, relative to a raw material monomer. When the ratio of these catalysts used is 0.01% by mass or more, sufficient activity may be obtained, and from the viewpoint of activity enhancement, the ratio of 10% by or less is sufficient and it is practical also in terms of economy.
The reaction is carried out in the presence of a solvent. As the solvent, those which are stable the above reaction conditions are used, and from the viewpoint of solubility of a raw material monomer, an solvent and an solvent are preferable. Specific examples include tetrahydrofuran, ethyl and the like. They may be singly or in a combination of two or more kinds.
A reaction product may be purified by distillation, crystallization, column-separation and the like, and the purification method may be selected depending on the nature of the products and the kind of impurities.

Synthesis method 2 is to react a non-aromatic adamantane with an alkyl-containing cyclic ether compound in the presence of a base catalyst to obtain a non-aromatic adamantane derivative in which a cyclic ether group is introduced.
Examples of the non-aromatic adamantanes include cyclohexyl-containing adamantane derivatives such as 1,3-bis(4-hydroxycyclohexyl)adamantane, 2,2-bis(4-hydroxycyclohexyl)adamantane and the like obtained by hydrogenating an adamantane bisphenol, cyclohexyl-containing adamantane derivatives such as 1,3-bis(4-aminocyclohexyl)adamantane, 2,2-bis(4-aminocyclohexyl)adamantane and the like obtained by hydrogenating an adamantane bisaniline, cyclohexyl-containing adamantane derivatives such as 1,3-bis(4-hydroxycarbonylcyclohexyl)adamantane, 2,2-bis(4-hydroxycarbonylcyclohexyl)adamantane and the like obtained by hydrogenating an adamantane bisaniline, and others.
Examples of the alkyl-containing cyclic ether compounds include a substituted alkyl-containing cyclic compound and the like represented by the following formula.

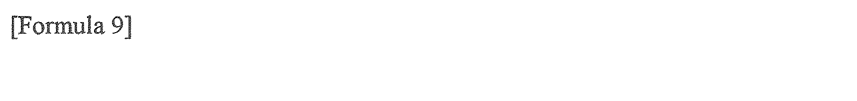

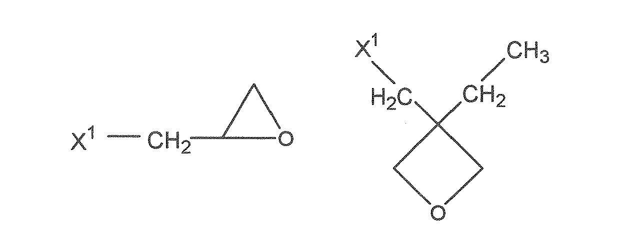

### [In the formula, X¹ represents Cl, Br, I, OTs (a tosyloxy group), or OMs (a mesyloxy group).]

A reaction between a non-aromatic adamantane and an alkyl-containing cyclic ether compound as mentioned above is carried out usually at a temperature of about 0 to about 200°C, and preferably at 20 to 150°C. When the reaction temperature is 0°C or above, the reaction rate does not decrease and remains moderate, thus the reaction time is shortened. When the reaction temperature is 200°C or below, product coloring may be suppressed. Applied absolute pressure at the reaction is about 0.01 to about 10 MPa, and preferably normal pressure to 1 Wa. When the reaction pressure is 10 MPa or less, special equipment is not necessary as the safety is secured, thus it is useful from an industrial viewpoint. The reaction time is usually about 1 minute to about 24 hours, and preferably 1 to 10 hours.

The above reaction is carried out in the presence of a catalyst. Examples of the base catalysts include sodium amide, triethylamine, tributylamine, trioctylamine, pyridine, N,N-dimethylaniline, 1,5-diazabicyclo[4.3.0]nonene-5 (DBN), 1,8-diazabicyclo[5.4.0]undecene-7 (DBU), tetramethylammonium chloride, tetraethylammonium chloride, sodium hydroxide, potassium hydroxide, sodium hydride, sodium phosphate, potassium phosphate, sodium carbonate, potassium carbonate, silver oxide, sodium methoxide, potassium t-butoxide and the like.
The ratio of a base catalyst used relative to a raw material is about 0. 8 to about 10, and preferably 1 to 5 in terms of a base catalyst/active hydrogen of a raw material monomer (molar ratio).
When carrying out the above reaction, quaternary ammonium salts such as tetramethylammonium chloride, tetraethylammonium bromide and the like may be added as a phase-transfer catalyst. The ratio of a quaternary ammonium salt used relative to a non-aromatic adamantane is 0.01 to 20 mol %, preferably 0.1 to 10 mol %.

The reaction is carried out in the absence or presence of a solvent. As the solvent, a solvent having 0.5% by mass or more, and preferably 5% by or more of solubility of the above non-aromatic adamantane in it is used. The amount of the solvent to be used is such that the concentration of the above non-aromatic adamantane in it is 0.5% by mass or more, and preferably 5% by mass or more. Here, the above-mentioned adamantane may exist in the of suspension but preferably in the of solution. Specific examples of the solvents include hexane, toluene, DMF (dimethylformamide), DMAc (N,N-dimethylacetamide), DMSO (dimethylsulfoxide), ethyl acetate, diethyl ether, tetrahydrofuran and the like. These may be used singly or in a combination of two or more kinds.
A reaction product may be purified by distillation, crystallization, column-separation and the like, the purification method may be selected depending on the nature of the products and the kind of impurities.

Synthesis method 3 is to carry out an addition reaction between a non-aromatic adamantane and a halohydrin compound under an acidic condition, followed by a ring-closure reaction in the presence of a catalyst to obtain an epoxy compound of a non-aromatic adamantane.
Examples of the non-aromatic adamantanes include cyclohexyl-containing adamantane derivatives such as 1,3-bis(4-hydroxycyclohexyl)adamantane, 2,2-bis(4-hydroxycyclohexyl)adamantane and the like obtained by hydrogenating an adamantane bisphenol.
As a halohydrin compound, the ones represented by the following general formula may be cited as examples.

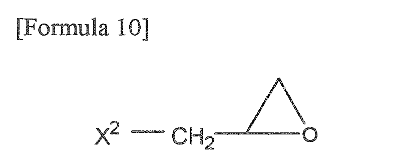

(In the formula, X² represents Cl, Br, or I.)
The addition reaction of a halohydrin is carried out usually at a temperature of about 0 to about 100°C, and preferably at 20 to 85°C. When the reaction temperature is 0°C or above, the reaction rate does not decrease and remains moderate, thus the reaction time is shortened. Further, when the reaction temperature is 100°C or below, the formation of a by-product derived from a halogen-containing substance may be suppressed. Applied absolute at the reaction is about 0.01 to about 10 MPa, preferably normal pressure to 1 MPa. When the reaction pressure is 10 MPa or less, equipment is not necessary as the safety is secured, thus it is useful from an industrial viewpoint. The reaction time is usually about 1 to about 24 hours, and preferably 30 minutes to 3 hours.
The above addition reaction is usually carried out in the presence of an acid catalyst. Examples of the acid catalysts include sulfuric acid, boron trifluoride, tin tetrachloride and the like.
The ratio of an acid catalyst used relative to a raw material monomer is about 0. 1 to about 20% by mol, and preferably 0.5 to 10% by mol. When the ratio of an acid catalyst used is 20% by mol or less, the formation of a by-product derived from a halogen-containing substance may be suppressed, and when it is 0.1% by mol or more, the reaction rate does not decrease and remains moderate, thus the reaction time is shortened.

The reaction is carried out in the absence or presence of a solvent. As the solvent, a solvent having 0.5% by or more, preferably 5% by mass or more of solubility of the above non-aromatic adamantane in it is used. The amount of the solvent to be used is such that the concentration of the above non-aromatic adamantane in it is 0.5% by mass or more, preferably 5% by or more. Here, the above-mentioned adamantane may exist in the state of suspension but preferably in the of solution. Specific examples of such solvents include hexane, heptane, toluene, D (dimethylformamide), DMAc (N,N-dimethylacetamide), DMSO (dimethylsulfoxide), ethyl acetate, diethyl ether, tetrahydrofuran and the like. These may be used singly or in a combination of two or more kinds.
In the synthesis method 3, the above-mentioned addition reaction is followed by a ring-closure reaction which is carried out in the presence of a catalyst. The ring-closure reaction is carried out usually at a temperature of about 20 to about 100°C, and preferably at 30 to 80°C. When the reaction temperature is 20°C or above, the reaction rate does not decrease and remains moderate, thus the reaction time is shortened. In addition, when the reaction temperature is 100°C or lower, a side reaction may be suppressed, thus chlorine content in an obtained adamantane derivative may be lowered. Applied absolute pressure at the reaction is about 0.01 to about 10 MPa, and preferably normal pressure to 1 MPa. When the reaction pressure is 10 MPa or lower, special equipment is not necessary as the safety is secured, thus it is useful from an industrial viewpoint. The reaction time is usually about 1 minute to about 24 hours, and preferably 30 minutes to 10 hours.

Examples of the base catalysts include sodium hydroxide, potassium hydroxide, sodium phosphate, potassium phosphate, sodium carbonate, potassium carbonate, calcium hydroxide, magnesium hydroxide and the like.
The amount of a base catalyst used, other than the amount consumed for neutralization of an acid catalyst used in the above addition reaction of a halohydrin compound, is 1 to 2 equivalents, and preferably 1 to 1.5 equivalents, relative to a hydroxyl group and a. carboxyl group of a raw material monomer. When the ratio of a catalyst used is 2 equivalents or less, the hydration reaction to give glycidyl ether is suppressed, when it is 1 equivalent or more, the ring-closure reaction to give a glycidyl ether sufficiently.
When a solvent is used in the addition reaction of a halohydrin compound, this solvent may be as it is. When the above addition reaction is carried out in the absence of a solvent, the solvent as above may be used.
Reaction products may be purified by distillation, crystallization, column-separation and the like, and the purification method may be selected depending on the nature of the product and the kind of impurities.
By further reacting a curing group of an intended compound with a raw material monomer without it from reaction products obtained in the above-mentioned synthesis methods 1 to 3, an epoxy resin having an adamantane skeleton, represented by the following general formula (V) or (VI), may be obtained.

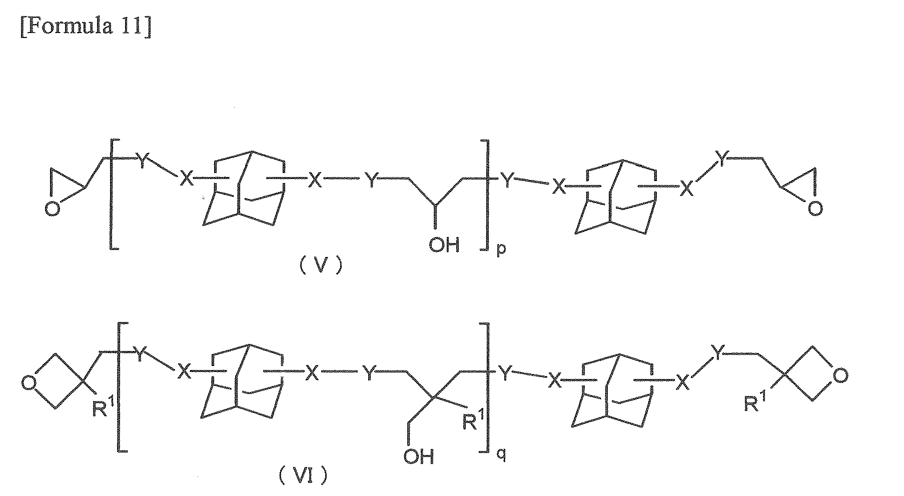

### [In the formula, X,Y, and R¹ are the same as the above-mentioned, and p and q an integer of 0 to 5.]

In an epoxy resin represented by the above formula (V) or (VI), the content of hydrolyzable chlorine in it is preferably 500 pp by or less.

A composition of the invention is composed of an adamantane derivative represented by the above formula (1) and/or an epoxy resin represented by the above general formula (V) or (VI), and a curing for an epoxy resin. In a resin composition of the present invention, in view of mechanical strength of a cured product, solubility of a resin composition, optimization of workability and the like, a resin mixture comprising an adamantane derivative represented by the above general formula (I) and/or an epoxy resin represented by the above general formula (V) or (VI) and other publicly known epoxy resins may also be used.
Examples of the publicly known epoxy resins include a bisphenol A epoxy resin, a bisphenol F epoxy resin (bisphenol A diglycidyl ether, bisphenol AD diglycidyl ether, bisphenol S diglycidyl ether, hydrogenated bisphenol A diglycidyl ether, bisphenol F diglycidyl ether, bisphenol G diglycidyl ether, tetramethylbisphenol A diglycidyl ether, bisphenol AF diglycidyl ether, bisphenol C diglycidyl ether and the like), novolak epoxy resins such as a phenol novolak epoxy resin, a cresol novolak epoxy resin and the like, an alicyclic epoxy resin, nitrogen-containing cyclic epoxy resins such as triglycidyl isocyanurate, a hydantoin epoxy resin and the like, a hydrogenated bisphenol A epoxy resin, an aliphatic epoxy resin, a glycidyl epoxy resin, a bisphenol S epoxy resin, a biphenyl-type epoxy resin and a dicyclo-type cyclic epoxy resin that are a mainstream of a low water-absorption curing type, a naphthalene epoxy resin, polyfunctional epoxy resins such as trimethylolpropane polyglycidyl ether, glycerol polyglycidyl ether, pentaerythritol polyglycidyl ether and the like, fluorine-containing epoxy resins such as a bisphenol AF epoxy resin, and the like, a (meth)acrylate diglycidyl ester, and others. Among them, an epoxy resin not having an aromatic ring is preferable. These may be singly or in a combination of two or more kinds,

The above publicly known epoxy resin may be solid or liquid at room temperature, but generally the having an epoxy equivalent of 120 to 2000 is preferable. When the epoxy equivalent is 120 or more, a cured epoxy resin composition is not brittle and has suitable strength. Also, when the epoxy equivalent is 2000 or less, the transition temperature (Tg) of the cured product thereof is not low and thus suitable.
In a resin mixture comprising an adamantane derivative represented by the above general formula (1) and/or an epoxy resin represented by the above general formula (V) or (VI) and the above publicly known epoxy resin, the content of the adamantane derivative represented by the above general formula (I) and/or the epoxy resin represented by the above general formula (V) or (VI) is preferably 5% by mass or more, and far preferably 10% by mass or more. When the content is 5% by ass or more, optical characteristics, long-term resistance, electric characteristics of a resin composition of the present invention become sufficient.

As a curing agent for an epoxy resin contained in a resin composition of the invention, at one kind selected from a cationic polymerization initiator, an acid anhydride type curing an amine type curing and a phenolic type curing may be cited. Namely, a resin composition of the invention may be cured by cationic polymerization using a cationic polymerization initiator, by a reaction using an acid anhydride type curing agent, an amine type curing and the like, or by a reaction using a cationic polymerization initiator and an acid anhydride type curing agent.
As the cationic polymerization initiators, those initiators which may react with an group or an oxetanyl group by heat or UV light may be used. Examples of such initiators include aromatic diazonium salts such as p-methoxybenzenediazonium hexafluorophosphate and the like; aromatic sulfonium salts such as triphenylsulfonium hexafluorophosphate and the like; aromatic iodonium salts such as diphenyliodonium hexafluorophosphate and the like; an aromatic iodosyl salt, an aromatic sulfoxonium salt, a metallocene compound and the like. Among them, aromatic sulfonium salts such as triphenylsulfonium hexafluorophosphate and the like, aromatic iodonium salts such as diphenyliodonium hexafluorophosphate and the like may be most suitable. They may be used singly or in a combination of two or more kinds.
The amount of the cationic polymerization initiator used is preferably 0.01 to 5.0 parts by mass, far preferably 0.1 to 3.0 parts by mass, relative to 100 parts by mass of the above adamantane derivative and/or the above epoxy resin, or the above resin mixture (hereinafter sometimes referred to as "Resin Component"). By choosing the content of the cationic polymerization initiator within the above range, suitable polymerization is achieved and good physical properties such as optical characteristics and the like may be expressed.

In the invention, as a curing an acid anhydride type curing agent, a phenolic type curing agent, an type curing and the like may be concurrently depending on the purpose. By reacting an adamantane derivative of the present invention having excellent heat resistance and transparency with a curing light resistance, dielectric constant and the like, in addition to heat and transparency, may be improved, and solubility for the practical use may be imparted.
Examples of the acid anhydride type curing agents phthalic anhydride, maleic anhydride, trimellitic anhydride, pyromellitic anhydride, haxahydrophthalic anhydride, tetrahydrophthalic anhydride, methylnadic anhydride, nadic anhydride, glutaric anhydride, methylhexahydrophthalic anhydride, methyltetrahydrophthalic anhydride and the like. Among them, haxahydrophthalic anhydride, tetrahydrophthalic anhydride, methylhexahydrophthalic anhydride, and methyltetrahydrophthalic anhydride are most suitable. They may be used singly or in a combination of two or more kinds.
When an acid anhydride type curing agent is used, a curing accelerator may be blended for the purpose to accelerate curing. Examples of such curing accelerators include a tertiary amine, an imidazole, an organic phosphine compound or its salt, metallic soap such as zinc octylate, tin octylate and the like. They may be used singly or in a combination of two or more kinds.

Examples of the phenolic type curing include a phenol novolak resin, a cresol novolak resin, a bisphenol A novolak resin, a triazine-modified phenol novolak resin and the like. Examples of the amine type curing include dicyandiamide and aromatic diamines such as m-phenylenediamine, 4,4'-diaminodiphenylmethane, 4,4'-diaminodiphenylsulfone, m-xylylenediamine and the like. They may be used singly or in a combination of two or more kinds.
Among curing an acid anhydride type curing agent is preferable in view of physical properties such as and the like of a cured and particularly haxahydrophthalic anhydride, tetrahydrophthalic anhydride, methylhexahydrophthalic anhydride, and methyltetrahydrophthalic anhydride are most preferable.

The blending ratio of a resin component and a curing agent is determined by the ratio of a glycidyl-reactive functional group of the curing agent. The ratio is usually 0.5 to 1.5 equivalents, and preferably 0.7 to 1.3 equivalents of the functional group of the corresponding curing agent, relative to one glycidyl equivalent. By choosing the blending ratio between the resin composition and the curing agent in the above range, there is no slowing of a curing rate of a resin composition, nor lowering of glass transition temperature of a cured resin and lowering of humidity resistance, thus it is suitable.

Further, in a resin composition of the present invention, many kinds of publicly known additives have been conventionally used may be blended as appropriate, if necessary. Examples of such additives include a curing accelerator, a decay-preventing agent, a modifying agent, a silane coupling agent, a defoaming agent, inorganic powders, a solvent, a leveling agent, a mold-release agent, a dye, pigments and the like.
As the above curing accelerator, there is no particular restriction, and examples of them include tertiary amines such as 1 ,8-diazabicyclo[5.4.0]undecene-7, triethylenediamine, tris(2,4,6-dimethylaminomethyl)phenol and the like; imidazoles such as 2-ethyl-4-methylimidazole, 2-methylimidazole and the like; phosphorous compounds such as triphenylphosphine, tetraphenylphosphonium bromide, tetraphenylphosphomum-tetraphenylborate, tetra-n-butylphosphonium-o,o-diethylphosphorodithioate the like; a quaternary ammonium salt, an organometallic salt, derivatives the like. These may be used singly or in a combination of two or more kinds. Among these curing accelerators, the use of a tertiary amine, an imidazole, and a phosphorous compound is preferable.
The content of the curing accelerator is preferably 0.01 to 8.0 parts by mass, and far preferably 0.1 to 3.0 parts by ass relative to 100 parts by mass of the above resin component. By choosing the content of the curing accelerator in the above range, sufficient curing accelerating effect may be obtained, and coloring of a cured product is not observed.

As a decay-preventing agent, examples of such publicly known decay-preventing include a phenolic compound, an amine compound, an organic sulfur compound, a phosphorous compound and the like. By adding a decay-preventing agent, such properties as heat-resistance, transparency and the like may be retained.
Examples of the phenolic compounds include commercially available materials such as Irganox 1010 (trademark, manufactured by Ciba Specialty Chemicals Inc.), Irganox 1076 (trademark, manufactured by Ciba Specialty Chemicals Inc.), Irganox 1330 (trademark, manufactured by Ciba Specialty Chemicals Inc.), Irganox 3114 (trademark, manufactured by Ciba Specialty Chemicals Inc.), Irganox 3125 (trademark, manufactured by Ciba Specialty Chemicals Inc.), Irganox 3790 (trademark, manufactured by Ciba Specialty Chemicals Inc.), BHT, Cyanox 1790 (trademark, Manufactured by Cyanamid Co.), Sumilizer GA-80 (trademark, manufactured by Sumitomo Chemical Co., Ltd.) and the like.

Examples of the amine compounds include such compounds as Irgastab FS042 (trademark, manufactured by Ciba Specialty Chemicals Inc.), GENOX EP (trademark, manufactured by Crompton Corp., chemical name: dialkyl-N-methylamine oxide) and the like; and such hindered amines as ADK STAB LA-52, LA-57, LA-62, LA-63, LA-67, LA-68, LA-77, LA-82, LA-87, and LA-94, all manufactured by Asahi Denka Co., Ltd., Tinuvin 123, 144, 440, 662, Chimassorb 2020, 119, and 944, all manufactured by Ciba Specialty Chemicals Inc., Hostavin N30 manufactured by Hoechst GmbH, Cyasorb UV-3346 and UV-3526, both manufactured by Cytec Industries Inc., Uval 299 manufactured by Great Lakes Chemical Corp., Sanduvor PR-31 manufactured by Clariant Corp. and the like.
Examples of the organic sulfur compounds include such commercially available products as DSTP (Yoshitomi) (trademark, manufactured by Yoshitomi Pharmaceutical Co., Ltd.), DLTP (Yoshitomi) (trademark, manufactured by Yoshitomi Pharmaceutical Co., Ltd.), DLTOIB (trademark, manufactured by Yoshitomi Pharmaceutical Co., Ltd.), DMTP (Yoshitomi) (trademark, manufactured by Yoshitomi Pharmaceutical Co., Ltd.), Seenox 412S (trademark, manufactured by Shipro Kasei, Ltd.), Cyanox 1212 (trademark, manufactured by Cyanamid Co.) and the like.

Examples of the modifying include publicly known modifying such as a glycol, a silicone, an alcohol and the like. Examples of the coupling agents include publicly known coupling such as a silane-type, a titanate-type and the like. Examples of the defoaming include publicly known defoaming agents such as a silicone type and the like. Examples of the inorganic powders include publicly known inorganic powders such as glass powders, silica powders, titania, zinc oxide, alumina and the like having a particle diameter of several nm to 10 µm depending on their use. Examples of the solvents that may be used for epoxy resin powders and as a diluent solvent for coating include aromatic solvents such as toluene, xylene the like, and ketone solvents such as methyl ethyl ketone, methyl isobutyl ketone, cyclohexanone and the like.

As a curing method for a resin composition of the present invention, there may be used, for example, a curing method in which the above resin component, a curing agent and/or a cationic polymerization initiator, and various additives are mixed, charged into a mold (resin mold) or formed to an intended shape by coating, then cured by heating or irradiating a UV beam. In the case of thermal curing, curing temperature is usually about 50 to about 200°C, and preferably 100 to 180°C. By choosing the temperature at 50°C or above, poor curing does not occur, nor do coloring and the like at 200°C or below. The curing time is dependent on a resin component, a curing agent, a curing accelerator, and an initiator to be used, but preferably 0.5 5 to 6 hours.
The irradiation strength of a UV beam is usually about 500 to about 5000 mJ/cm², and preferably 1000 to 4000 mJ/cm². may be performed after the UV beam irradiation, preferably at 70 to 200°C for 0.5 to 12 hours.
Molding methods may be an injection molding, a blow molding, a molding and the like, and not particularly restricted, but an injection molding, by using a resin composition in pellet form in injection molding equipment, may be preferably used.

A obtained by curing a composition of the invention is excellent in resistance and transparency, wherein 70% or more of the total light transmittance may be attained. In addition, as is shown in the following the cured resin product having high glass transition temperature, excellent durability (in heat resistance and light resistance), and good electric characteristics such as dielectric constant and the like since it is excellent in processability of low melting point.
As is in the above, a resin composition of the present invention has excellent characteristics, thus is suitably used as a resin (a sealant and an adhesive) for an optical semiconductor (an LED and the like), a flat panel display (an organic EL device, a liquid crystal and the like), an electronic circuit, an optical circuit (an optical waveguide), and optical electronic members such as a lens for optical communication, an optical film and the like.

Therefore, a resin composition of the present invention can be used for a semiconductor element/an integrated circuit (an IC and the like), an individual semiconductor (a diode, a transistor, a thermistor and the like), an LED (an LED lamp, a chip LED, a light receiving element, and a lens for an optical semiconductor), a sensor (a temperature sensor, a. light sensor, and a magnetic sensor), a passive component (a high frequency device, a resistor, a condenser and the like), a structural component (a connector, a switch, a relay and the like), an automobile part (a circuit system, a control system, sensors, a lamp seal and the like), an adhesive (an optical component, an optical disk, a pickup lens) and the like, and, in addition, for an optical film and the like as surface coating.
Therefore, the present invention also provides a sealant, made of the above-mentioned resin composition, for an optical semiconductor, optical electronic members such as an optical waveguide, a for optical communication, a sealant for an organic EL device, an optical film and the like, and a for an electronic circuit.

A composition as a sealant for an optical semiconductor (LED and the like) may be applied to a bombshell type device or a surface mount type (SMT) device and the like, may adhere well with semiconductors such as GaN formed on a metal or a polyamide, and further may be used by dispersing fluorescent dyes such as YAG and the like in it. Further, it may be used also for a surface coating material of a bombshell type LED and for a lens of a SMT type LED and the like.
A composition for an organic EL is applicable to the organic EL device having a composition of anode/hole-injection layer/luminescent layer/electron-injection layer/cathode which is formed in this order on the transparent substrates such as generally used glasses, transparent resins and the like. As a sealant for an organic EL device, it may be used as an adhesive to cover an EL device by a resin film coated with a metal can, a metal sheet, or SiN and the like, or may directly seal an EL device by dispersing inorganic fillers and the like in order to impart a gas-barrier property to a composition of the present invention. It may be applied to a bottom emission type, which is currently a mainstream as a display system, but the of transparency and heat resistance of a resin composition of the present invention may be advantageously utilized when applied to a top emission type, which will draw attention in view of the light extraction efficiency the like in the future.
A composition for an electronic circuit is applicable as an interlayer insulation film, as an adhesive between a polyimide for a flexible printed board and a copper foil, or as a resin for a substrate.
A composition for an optical circuit is applicable to a thermooptic switch and an arrayed waveguide grating for a single-mode and a multi-mode, an optical multiplexer/demultiplexer, a wavelength-variable filter, or a core material and a clad material for an optical fiber. It is also applicable to a micro lens array which focuses a light to a waveguide and a mirror of an MEMS-typc optical switch. Furthermore, it is applicable also to a dye binder and the like for a photoelectric transducer.
A composition for an optical film is applicable as a display of film substrates for liquid crystal and for organic EL, or as a light diffusion film, an anti-reflection film, a color-converting film using dispersion of a fluorescent dye and the like, and others.

### Examples

Next, the present invention will be explained in further detail, but the invention is not restricted at all by these Examples. In the following Examples and Comparative Examples, the resin compositions obtained are evaluated in the following manner.
(1) Glass transition temperature
   By using a differential scanning calorimeter (DSC-7, manufactured by PerkinElmer, Inc.), 10 mg of a sample was kept at 50°C for 5 minutes under nitrogen atmosphere and then heated at 10°C/minute. A discontinuous point observed in the thermal flux curve thereby obtained was taken as a transition temperature Tg.
(2) Light transmittance
   A 5-mm thickness specimen of a sample was measured in accordance with the procedure in JIS K7105 at the wavelength of 400 nm (unit %). A spectrophotometer UV-3100S, manufactured by Shimadzu Corporation, was used as the measuring instrument.
(3) Light resistance test
   By using Suntest CPS+, manufactured by Toyo Seiki Seisaku-Sho, Ltd., a sample was irradiated at 60°C by light for 500 hours, and the change of the light transmittance at 400 nm before after the irradiation was measured by using a sunshine tester. When the transmittance was decreased by less than 20%, it was rated as "good", and when decreased by 20% or more, it was rated as "poor".
(4) Long-term heat resistance test
   The change of the light transmittance at 400 nm of a sample before and after it was kept in an oven controlled at 140°C for 100 hours was measured by a sunshine tester. When the transmittance was decreased by less than 20%, it was rated as "good", and when decreased by 20% or more, it was rated as "poor".

### Example 1

### (1) Synthesis of 1,3-bis(4-glycidyloxycyclohexyl)adamantane

In an autoclave having an inner volume of 100 ml were charged 8.0 g of 1,3-bis(4-glycidyloxyphenyl)adamantane (227 epoxy equivalents), 0.5 g of a rhodium catalyst (manufactured by N.E. Chemcat Corp., trade name 5% Rh Carbon), and 20 g of tetrahydrofuran, and the atmosphere of the was replaced with hydrogen gas. The reaction was carried out for about 5 hours with stirring at 50°C under the hydrogen pressure of 4 a until lowering of the hydrogen stopped. After the reaction, the catalyst was removed by filtration and then the solvent by distillation to obtain the product, 1,3-bis(4-glycidyloxycyclohexyl)adamantane, shown by the following formula.

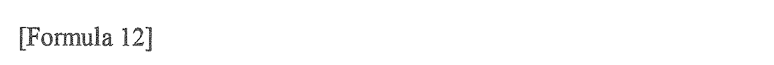

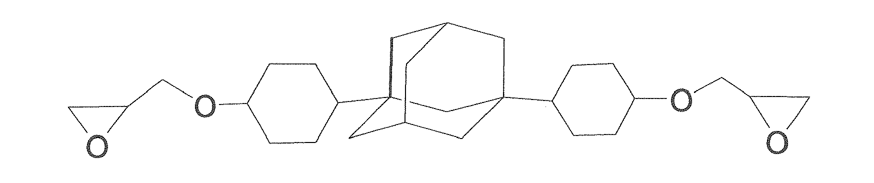

The ratio of ring-hydrogenation in the product was 99% as measured by the decrease of a UV absorbance (at the wavelength of 275 nm). The epoxy equivalent of the product was 264 and the ratio of the remaining epoxy was 88%.
The obtained product was identified by nuclear magnetic resonance spectra ('H-NMR and ¹³C-NMR). The nuclear magnetic resonance spectra were measured in CDCl₃ solvent by -ECA500 manufactured by JEOL Ltd. In the following, a number shows a position of a carbon atom and a hydrogen atom which is bonded to a carbon atom, when hydrogen atoms which are bonded to the same carbon atom are detected at two different positions, of them is by an apostrophe.

¹H-NNM (500 MHz): 0.81 (m, 2H, (10), (14)), 1.21 (s, 2H, (17)), 1.22-1.31 (m, 8H, (5), (6)), 1.31-1.37 (m, 4H, (9), (11), (13), (15)), 1.41-1.50 (m, 8H, (6)', (9)', (11)', (13)', (15)'), 1.52 (s, 2H, (16)), 1.91-2.00 (m, 6H, (5)', (7)), 2.59 (dd, 2H, (1)), 2.75 (t, 2H, (1)'), 3.09-3.13 (m, 2H, (2)), 3.37 (dd, 2H, (3)), 3.56 (m, 2H, (4)), 3.60 (dd, 2H, (3)'). ¹³C-NMR (127 MHz): 20.0 ((6)), 29.4 ((7)), 30.5, 30.8 ((5)), 35.1 ((8), (12)), 37.4 ((16)), 39.5 ((9), (11), (13), (15)), 42.4 ((17)), 44.6 ((1)), 48.5 ((10), (14)), 51.4 ((2)), 68.5 ((3)), 74.0 ((4)).

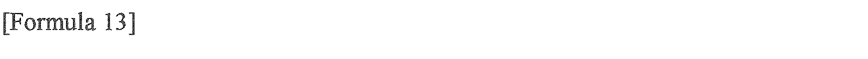

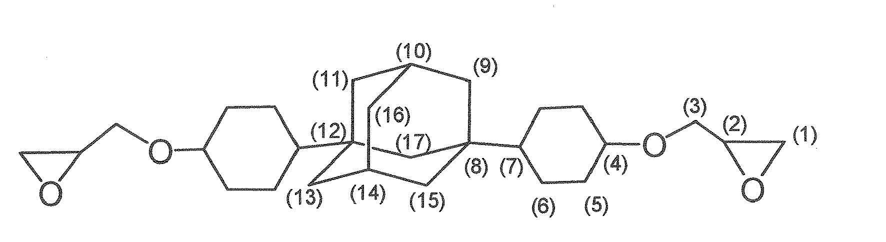

### (2) Production of a cured resin

A batch containing 1g of 1,3-bis(4-glycidyloxycyclohexyl)adamantane (264 epoxy equivalents) obtained in the above-mentioned (1), 0.64 g of methylhexahydrophthalic anhydride (manufactured by New Japan Chemical Co., Ltd., trade name MH700) as an acid anhydride, and 0.01g of 1,8-diazabicyclo[5.4.0]undecene-7 (manufactured by San-Apro Ltd., trade name SA102) as a curing accelerator was blended at room temperature, defoamed, then heated at 120°C for 2 hours and then at 150°C for 2 hours to prepare a cured resin (a sheet of 3mm thickness). A glass transition temperature and light transmittance of the cured resin product thus obtained were measured, and then it was subjected to the tests for light resistance long-term heat resistance. The evaluation results are shown in Table 1.

### Example 2

### (1) Synthesis of 2,2-bis(4-glycidyloxycyclohexyl)adamantane

In an autoclave having an inner volume of 100 ml were charged 8.0 g of 2,2-bis(4-glycidyloxyphenyl)adamantane (227 epoxy equivalents), 0.5 g of a rhodium catalyst (manufactured by N.E. Chemcat Corp., trade name 5% Rh Carbon), and 20 g of THF, and the atmosphere of the was with hydrogen gas. The reaction was carried out for about 5 hours with stirring at 50°C under the hydrogen pressure of 4 a until lowering of the hydrogen pressure stopped. After the reaction, the catalyst was removed by filtration and then the solvent by distillation to obtain the intended product, 2,2-bis(4-glycidyloxycyclohexyl)adamantane, shown by the following formula.

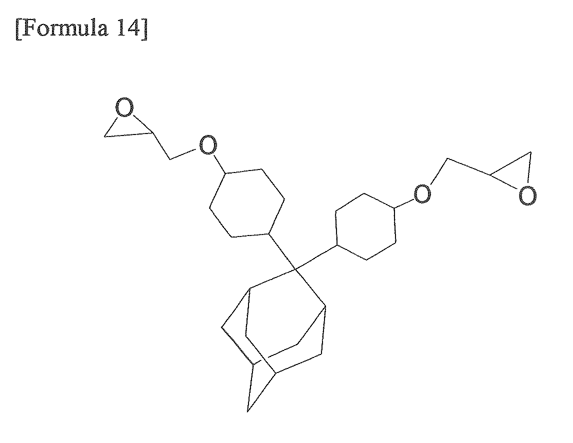

The ratio of ring-hydrogenation in the product was found to be 98% as measured by the decrease of a UV absorbance (at the wavelength of 275 nm). The epoxy equivalent of the product was 258 and the ratio of the remaining epoxy was 90%.

### (2) Production of a cured resin

A batch of 1 g of 2,2-bis(4-glycidyloxycyclohexyl)adamantane (258 epoxy equivalents) obtained in the above-mentioned (1), 0,65 g of methylhexahydrophthalic anhydride (manufactured by New Japan Chemical Co., Ltd., trade name 700) as an acid anhydride, and 0.01 g of 1,8-diazabicyclo[5.4.0]undecene-7 (manufactured by San-Apro Ltd., trade name SA102) as a curing accelerator was blended at room temperature, defoamed, and then heated at 120°C for 2 hours and then at 150°C for 2 hours to prepare a cured resin (a sheet of 3mm thickness). A transition temperature and light transmittance of the cured resin product thus obtained were measured, and then it was subjected to the tests for light resistance and long-term heat resistance. The evaluation results are shown in Table 1.

### Comparative Example 1

A cured product was prepared in the same manner as Example 1 (2), that BPA (bisphenol A) epoxy resin (185 epoxy equivalents), instead of 1,3-bis(4-glycidyloxycyclohexyl)adamantane, and 0.91 g of methylhexahydrophthalic anhydride were used, and a similar evaluation was carried out. The evaluation results are shown in Table 1.

### Comparative Example 2

A cured product was prepared in the manner as Example 1 (2), that a hydrogenated BPA epoxy resin (204 epoxy equivalents), instead of 1,3-bis(4-glycidyloxycyclohexyl)adamantane, and 0.82 g of methylhexahydrophthalic anhydride were used, and a similar evaluation was carried out. The evaluation results are shown in Table 1.

### Comparative Example 3

A batch containing 1 g of 1,3-bis(4-glycidyloxyphenyl)adamantane (227 epoxy equivalents) and 0.74 g of methylhexahydrophthalic anhydride was attempted for mixing, but was not dissolved at room temperature, thus the batch was heated to 70°C for mixing. To the mixture was added 0.01 g of 1,8-diazabicyclo[5.4.0]undecene-7 (manufactured by San-Apro Ltd., trade name SA102) as a curing accelerator, defoamed, and then heated at 120°C for 2 hours and 150°C for 2 hours to prepare a cured resin (a sheet of 3mm thickness). A glass transition temperature and light transmittance of the cured resin product thus obtained were measured, and then it was subjected to the tests for light resistance and long-term resistance. The evaluation results are shown in Table 1 .

### Comparative Example 4

A batch containing 1g of 1,3-bis(4-glycidyloxyphenyl)adamantane (227 epoxy equivalents) and 0.74 g of methylhexahydrophthalic anhydride was attempted for mixing, but was not dissolved at room temperature, thus the batch was heated to 80°C for mixing. The mixture was added by 0.01 g of 1,8-diazabicyclo[5.4.0]undecene-7 (manufactured by San-Apro Ltd., trade name SA102) as a curing accelerator, defoamed, and then heated at 120°C for 2 hours and at 150°C for 2 hours to prepare a cured resin (a sheet of 3mm thickness). A transition temperature and light transmittance of the cured product thus obtained were measured, and then it was subjected to the for light and long-term resistance. The evaluation results are shown in Table 1.

[Table 1]

**[Table 1]**

| | Example 1 | Example 2 | Comparative Example 1 | Comparative Example 2 | Comparative Example 3 | Comparative Example 4 |
|---|---|---|---|---|---|---|
| Resin dissolving temperature (°C) | Room temperature | Room temperature | Room temperature | Room temperature | 70 | 70 |
| Glass transition temperature (°C) | 145 | 145 | 130 | 105 | 170 | 170 |
| Light transmittance (400 mn, %) | 92 | 92 | 80 | 92 | 82 | 85 |
| Light resistance test | good | good | poor | good | poor | poor |
| Heat resistance test | good | good | good | poor | good | good |

### Industrial Applicability

A composition containing an adamantane derivative and an epoxy resin having an adamantane skeleton of the invention gives a cured product that is excellent in optical characteristics such as transparency and light resistance, long-term heat resistance, and electric characteristics such as dielectric constant and the like, and is useful as a sealant for an optical semiconductor, an optical waveguide, a lens for optical communication, a sealant for an organic EL device, optical electronic such as an optical film the like, and a sealant for an electronic circuit. It is also useful as an organic EL device, displays such as a liquid crystal and the like, an illumination device using LED and the like, a coating material for information communication such as an optical circuit and the like, a sealant, an adhesive the like.

## Claims

1. An adamantane derivative represented by the following general formula (I): 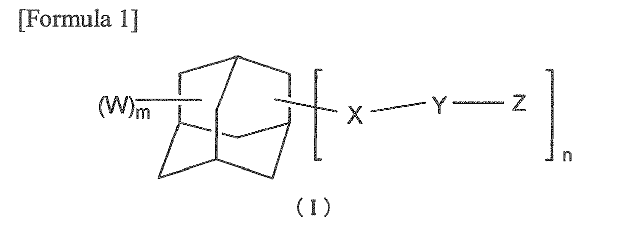 wherein W represents a group selected from a hydrogen atom, a halogen atom, a hydrocarbon group, a halogen-substituted hydrocarbon group, a cyclic hydrocarbon group, a halogen-substituted cyclic hydrocarbon group, a hydroxyl group, a carboxyl group, and =O formed from two Ws combined together; X represents a group represented by the following general formula (II): 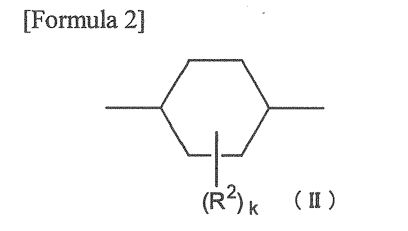 wherein R² an alkyl group having 1 to 4 carbon atoms; Y represents a group selected from -CO₂-, -O-, -N(R³)- and -N(Z)- wherein R*³ a hydrogen or an alkyl group 1 to 4 carbon atoms, and Z represents a group represented by the following general (III) or (IV): 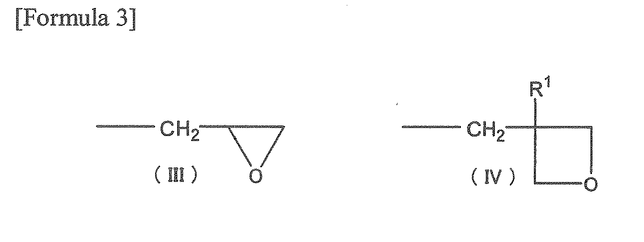 wherein R¹ represents a methyl group or an ethyl group; k represents an of 0 to 10; m represents an integer of 0 to 14; n represents an integer of 2 to 16; and +n= 16.

2. The adamantane derivative according to claim 1, wherein X is bonded to the bridgehead position of the adamantane skeleton and n is 2 to 4 in the general formula (1).

3. The adamantane derivative according to claim 1, wherein n is 2 and X is bonded to the same methylene position of the adamantane skeleton in the general formula (I).

4. The adamantane derivative according to claim 2 or claim 3, wherein Y is -O- in the formula (I).

5. The adamantane derivative according to any of the claim 1 to 4, wherein the content of hydrolyzable chlorine is 500 pp by mass or less.

6. An epoxy resin having an adamantane skeleton represented by the following general formula (V) or (VI): 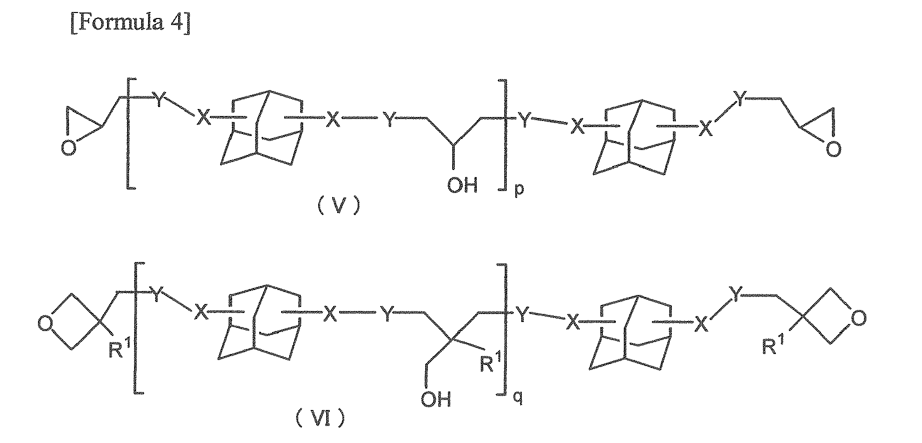 wherein X represents a group represented by the following general formula (II): 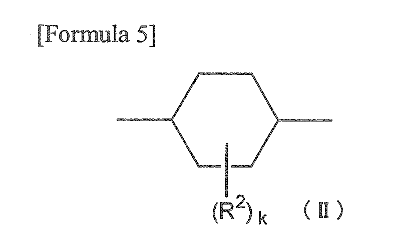 wherein R² represents an alkyl group having 1 to 4 carbon atoms, Y represents a group selected from -CO₂-, -O-, -N(R³)- and -N(Z)- wherein R³ represents a hydrogen atom or an alkyl group having 1 to 4 carbon atoms; R¹ represents a methyl group or an ethyl group; k represents an integer of 0 to 10; p and q represent an integer of 0 to 5.

7. The epoxy according to claim 6, wherein the content of hydrolyzable chlorine is 500 ppm by mass or less.

8. A method for producing the adamantane derivative according to any of claims 1 to 5, wherein a corresponding aromatic adamantane derivative is ring-hydrogenated in the presence of a rhodium catalyst or a ruthenium catalyst.

9. A resin composition containing at any one of the adamantane derivative according to any of the claim 1 to 5 and/or the epoxy resin to claim 6 or 7, and a curing for an epoxy resin.

10. The resin composition to claim 9, wherein the curing agent for an epoxy resin is at any one of a cationic polymerization and/or an acid anhydride type curing agent.

11. An optical electronic member the resin composition according to claim 9 or 10.

12. A for an electronic circuit the composition according to claim 9 or 10.
